# EUROPEAN PATENT APPLICATION

(11) **EP 2 774 649 A2**
(43) Date of publication of application: **10.09.2014**
(21) Application number: 14155177.0
(22) Date of filing: 14.02.2014
(51) Int. Cl.: A61M 25/10

(54) **Balloon catheter inflation regulator**

(30) Priority: 04.03.2013 US 201313783447
(71) Applicant: Covidien LP, Mansfield, MA 02048 (US)
(72) Inventor: Malhi, Arnaz, Watertown, MA Massachusetts 02472 (US)
(74) Representative: Gray, James

(57) **Abstract**

A system 1000 for use in a medical procedure includes a catheter 100 defining a balloon lumen, at least one balloon 112 that is secured to an outer surface of the catheter, and a regulator 200 that is at least partially disposed within the balloon lumen. The regulator includes proximal 204 and distal end 206 portions, and defines a passage 210 extending from the proximal end portion to the distal end portion. The distal end portion of the regulator includes an outer surface defining at least one opening 216 that is in fluid communication with the passage. The at least one opening of the regulator is movable within the balloon lumen to control fluid communication between the passage and the at least one balloon.

## Description

### TECHNICAL FIELD

The present disclosure relates to the treatment of a patient's vasculature, and, more specifically, relates to balloon catheter inflation.

### BACKGROUND

Certain varieties of catheters include expandable structures, such as inflatable balloons. In general, during the use of such catheters, fluid is communicated into the inflatable balloons to achieve a particular effect. For example, in some instances, fluid is communicated into inflatable balloons, to secure the catheter in a targeted location, or to arrange the catheter, or a portion thereof, in a particular orientation relative to the vasculature or relative to an additional medical device utilized during treatment. In catheters having multiple different inflatable balloons, separate lumens are used for each balloon to permit independent expansion of the balloons relative to one another.

### SUMMARY

In one aspect of the present disclosure, a system for use in a medical procedure includes a catheter defining a balloon lumen, at least one balloon secured to an outer surface of the catheter, and a regulator at least partially disposed within the balloon lumen. The regulator includes proximal and distal end portions, and defines a passage extending from the proximal end portion to the distal end portion. The distal end portion of the regulator has an outer surface defining at least one opening in fluid communication with the passage. The at least one opening of the regulator is movable within the balloon lumen to control fluid communication between the passage and the at least one balloon. For example, the at least one opening may be movable along a longitudinal axis of the balloon lumen and/or rotatable about the longitudinal axis of the balloon lumen.

A portion of the catheter defining the balloon lumen may be dimensioned to form a substantially fluid-tight seal with a portion of the regulator adjacent the at least one opening.

The at least one opening may include a plurality of openings, which may be axially spaced from one another along the longitudinal axis of the balloon lumen of the catheter and/or circumferentially spaced from one another along the outer surface of the regulator.

The at least one balloon may span a circumference of the outer surface of the catheter body. Proximal and distal balloons may be provided that are axially spaced from one another along the longitudinal axis of the balloon lumen. In one aspect, the at least one balloon may include a first balloon and a second balloon, and the at least one opening may be movable within the balloon lumen to establish fluid communication between the passage of the regulator and one of the first and second balloons while fluidly isolating the passage of the regulator from the other one of the first and second balloons.

The distal end portion of the regulator may include a closed end distal to the at least one opening. The regulator may include an outer, transverse cross-section that is uniform from the proximal end portion to the distal end portion. The regulator may also include an outer, transverse cross-section that is largest adjacent the at least one opening.

The catheter may further define a main lumen that is substantially parallel to the balloon lumen. The main lumen may define a transverse cross-sectional area larger than a transverse cross-sectional area defined by the balloon lumen.

The outer surface of the catheter may define at least one orifice in fluid communication with the at least one balloon, and the at least one opening of the regulator may be movable within the balloon lumen to control fluid communication between the passage and the at least one orifice defined by the outer surface of the catheter.

In another aspect of the present disclosure, methods are disclosed for controlling inflation of a balloon catheter. A method includes positioning at least a distal end portion of a regulator within a balloon lumen defined by a catheter, introducing fluid into a passage defined by the regulator, and moving the at least one opening of the regulator within the balloon lumen to control fluid communication between the passage and the at least one balloon. The distal end portion of the regulator has an outer surface that defines at least one opening in fluid communication with the passage. The passage extends from the proximal end portion of the regulator to the distal end portion of the regulator, and the fluid is introduced into a proximal end portion of the passage defined by the regulator.

Moving the at least one opening of the regulator may include aligning the at least one opening with at least one orifice defined by an outer surface of the catheter in fluid communication with the balloon such that fluid introduced into the passage of the regulator flows into a volume defined by the balloon. Aligning the at least one opening with the at least one orifice defined by the outer surface of the catheter may include rotating the regulator about a longitudinal axis of the balloon lumen. Moving the at least one opening of the regulator may include misaligning the at least one opening and the at least one orifice to inhibit the flow of fluid from the passage of the regulator into the volume defined by the balloon.

The method may additionally or alternatively include measuring a pressure of the fluid introduced into the proximal end portion of the passage of the regulator, wherein moving the at least one opening of the regulator is based at least in part on the measured pressure of the fluid.

Embodiments of the present disclosure can include one or more of the following advantages.

Known catheters including multiple inflatable balloons and employing separate lumens for each balloon can be complex in design, more costly to manufacture, and subject to an increased rate of failure during use, e.g., due to operator error. A catheter including multiple inflatable balloons with a simpler design would therefore be advantageous so as to reduce complexity in design, and thus, the cost of manufacture, while increasing ease of use.

Other aspects, features, and advantages of the presently disclosed subject matter will be apparent from the description, the drawings, and the claims.

### BRIEF DESCRIPTION OF THE DRAWINGS

FIG. 1 is a schematic representation of a balloon catheter system for use in a medical procedure.
FIG. 2 is a perspective view of the balloon catheter system of FIG. 1, with balloon members of the catheter shown in an initial unexpanded condition.
FIG. 3 is a transverse, cross-sectional view of the balloon catheter system taken through lines 3-3 in FIG. 2.
FIG. 4 is a perspective view of a catheter of the balloon catheter system of FIG. 1, with balloon members in an at least partially expanded condition.
FIG. 5 is a perspective view of a regulator of the balloon catheter system of FIG. 1.
FIG. 6 is a transverse, cross-sectional view of the regulator of FIG. 5 taken through lines 6-6 in FIG. 5.
FIGS. 7A-7D are transverse, cross-sectional views of the balloon catheter system of FIG. 1 with the regulator shown in different positions within the catheter.
FIG. 8 is a perspective view of a regulator of a balloon catheter system.
FIG. 9 is a transverse, cross-sectional view of the regulator of FIG. 8 taken through lines 9-9 in FIG. 8.
FIG. 10 is a perspective view of a regulator of useable with the balloon catheter system of FIG. 1, with the regulator shown positioned within the catheter.

### DETAILED DESCRIPTION

Embodiments of the present disclosure will now be described in detail with reference to the drawings, wherein like reference numerals identify similar or identical elements. As used herein, the term "distal" refers to that portion of a device, or a component thereof, furthest from the user, such as a clinician or physician, during proper use. The term "proximal" refers to that portion of a device, or a component thereof, closest to the user during proper use. Additionally, the term "vasculature" includes any passage or channel, either natural or artificial, within the body. Examples of such passages or channels include a blood vessel, a blood vessel graft, and a fistula.

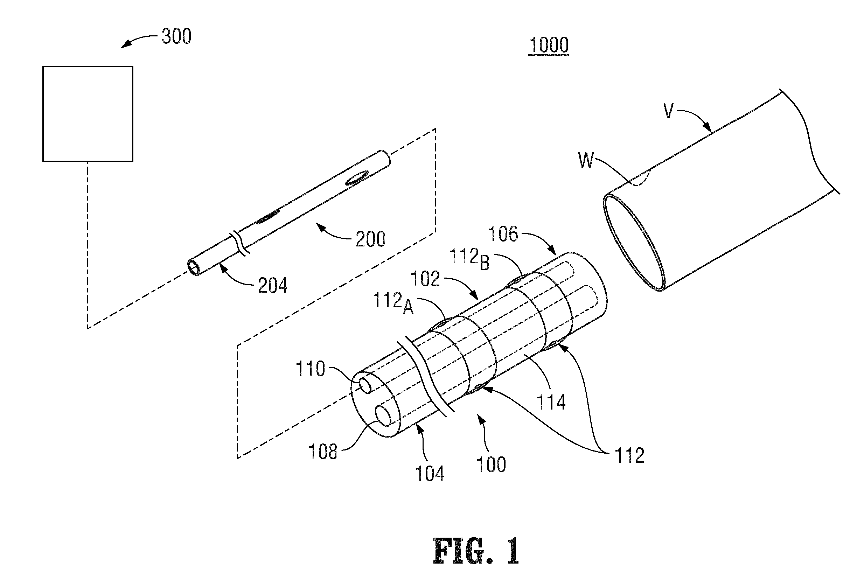

Referring now to FIG. 1, a balloon catheter system 1000, useful in the treatment of a patient's vasculature, includes a catheter 100, a regulator 200 that is insertable into the catheter 100, and a fluid source 300 in fluid communication with the regulator 200.

With reference now to FIGS. 1-4, the catheter 100 includes an elongated catheter body 102 having respective proximal and distal end portions 104, 106, and defining a first longitudinal axis X_{C} extending from the proximal end portion 104 to the distal end portion 106. The catheter 100 may be formed, such as by extrusion, from one or more biocompatible materials sufficiently pliable to facilitate manipulation of the catheter 100 with respect to the blood vessel V, for example.

The catheter body 102 defines a main lumen 108 sized to receive a surgical instrument such as, for example, a thrombectomy catheter (not shown), and a balloon lumen 110. The lumens 108, 110 each extend between the respective proximal and distal end portions 104, 106 of the catheter body 102 in parallel relation to each other and to the longitudinal axis X_{C} of the catheter 100. The balloon lumen 110 defines longitudinal axis X_{B}, parallel to and radially offset from the first longitudinal axis X_{C}.

One or more balloon members 112 are secured to an outer surface 114 of the catheter body 102. While the catheter 100 is shown as including a pair of balloons 112_{A}, 112_{B}, it should be appreciated that the number of balloon members 112 included on the catheter 100 may be varied dependent, for example, upon the particular requirements of the procedure in which the catheter 100 is used.

The main lumen 108 defines a cross-sectional dimension D_{L1}, and the balloon lumen 110 defines an inner cross-sectional dimension D_{L2}. Each cross-sectional dimension D_{L1}, D_{L2} extends transverse to the longitudinal axis X_{C} of the catheter 100. The cross-sectional dimension D_{L1} defined by the main lumen 108 is shown as being larger than the cross-sectional dimension D_{L2} defined by the balloon lumen 110. In certain embodiments, however, the cross-sectional dimension D_{L1} defined by the main lumen 108 may be less than, or equal to, the cross-sectional dimension D_{L2} defined by the balloon lumen 110.

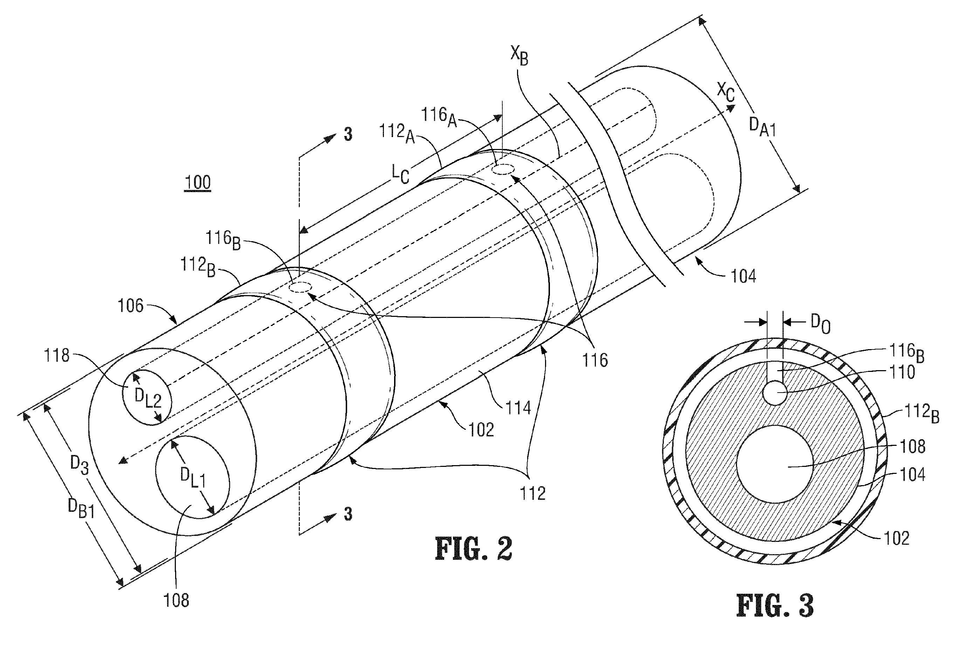

The outer surface 114 of the catheter body 102 is generally smooth to facilitate manipulation of the catheter 100 within the patient's vasculature, e.g., the blood vessel V (FIG. 1). The catheter body 102 defines one or more orifices 116, each corresponding in axial location to a respective balloon member 112. For example, defines a first orifice 116_{A} and a second orifice 116_{B}. The first orifice 116_{A} is in fluid communication with an interior volume defined by the balloon 112_{A}, and the second orifice 116_{B} is in fluid communication with an interior volume defined by the balloon 112_{B}. Although the orifices 116_{A}, 116_{B} are shown, in FIG. 2, as being in axially aligned parallel to the longitudinal axis X_{B} of the balloon lumen 110, in alternative configurations of the catheter 100, the orifices 116_{A}, 116_{B} may be circumferentially spaced from one another such that the orifices 116_{A}, 116_{B} are not axially aligned parallel to the longitudinal axis X_{B} of the balloon lumen 110.

The catheter 100 is shown as including a pair of orifices 116, with the orifice 116_{A} in fluid communication with the balloon 112_{A} and the orifice 116_{B} in fluid communication with the balloon 112_{B}. It should be appreciated, however, that the number of orifices 116 defined by the catheter body 102 may be varied. For example, the interior volume defined by each of the balloon members 112_{A}, 112_{B} may be in fluid communication with a plurality of orifices 116.

The orifices 116_{A}, 116_{B} each define a cross-sectional dimension Do, and are spaced apart from one another along the longitudinal axis X_{C} of the catheter 100 by an axial distance Lc generally corresponding to an axial distance between centers of the balloons 112_{A}, 112_{B}. The orifices 116_{A}, 116_{B} are each in fluid communication with the balloon lumen 110 such that fluid introduced into the balloon lumen 110 (e.g., from the fluid source 300), in the absence of the regulator 200, can exit the balloon lumen 110 and enter the balloons 112_{A}, 112_{B} through the respective orifices 116_{A}, 116_{B}.

The balloons 112_{A}, 112_{B} are formed from biocompatible material, and each balloon 112_{A}, 112_{B} may be compliant, semi-compliant, or noncompliant..

The balloons 112_{A}, 112_{B} circumscribe the outer surface 114 of the catheter body 102.

The balloon members 112_{A}, 112_{B} are spaced along the longitudinal axis X_{C} of the catheter 100 to encompass the respective orifices 116_{A} and 116_{B} defined by the catheter body 102. Dependent upon the particular requirements of the procedure in which the catheter 100 is used, the axial spacing between the balloons 112_{A}, 112_{B} may be altered or varied.

The balloons 112_{A}, 112_{B} are each movable between an uninflated condition and an at least partially inflated condition (e.g., compare FIG. 2 to FIG. 4). In the uninflated condition, the balloons 112_{A}, 112_{B} respectively define first cross-sectional dimensions D_{A1}, D_{B1} transverse to the longitudinal axis X_{C} of the catheter 100. The transverse cross-sectional dimensions D_{A1}, D_{B1} respectively defined by the balloon members 112_{A}, 112_{B} in the uninflated condition may correspond to an outer cross-sectional dimension D₃ defined by the catheter body 102 to facilitate insertion of the catheter 100 into the blood vessel V and/or to facilitate movement of the catheter 100 through the blood vessel V. In the at least partially inflated condition the balloons 112_{A}, 112_{B} respectively define second cross-sectional dimensions D_{A2}, D_{B2} transverse to the longitudinal axis X_{C} of the catheter 100. The second cross-sectional dimensions D_{A2}, D_{B2} are larger than the respective first cross-sectional dimensions D_{A1}, D_{B1}. The second cross-sectional dimensions D_{A2}, D_{B2} respectively defined by the inflated balloons 112_{A}, 112_{B} facilitate engagement of the balloons 112_{A}, 112_{B} with an internal wall W of the blood vessel V. Such engagement of the balloons 112_{A}, 112_{B} can, for example, maintain the catheter 100 in a particular location/orientation in the blood vessel V. As discussed in further detail below, the balloon members 112_{A}, 112_{B} may be moved between the uninflated condition and the inflated condition independently or simultaneously.

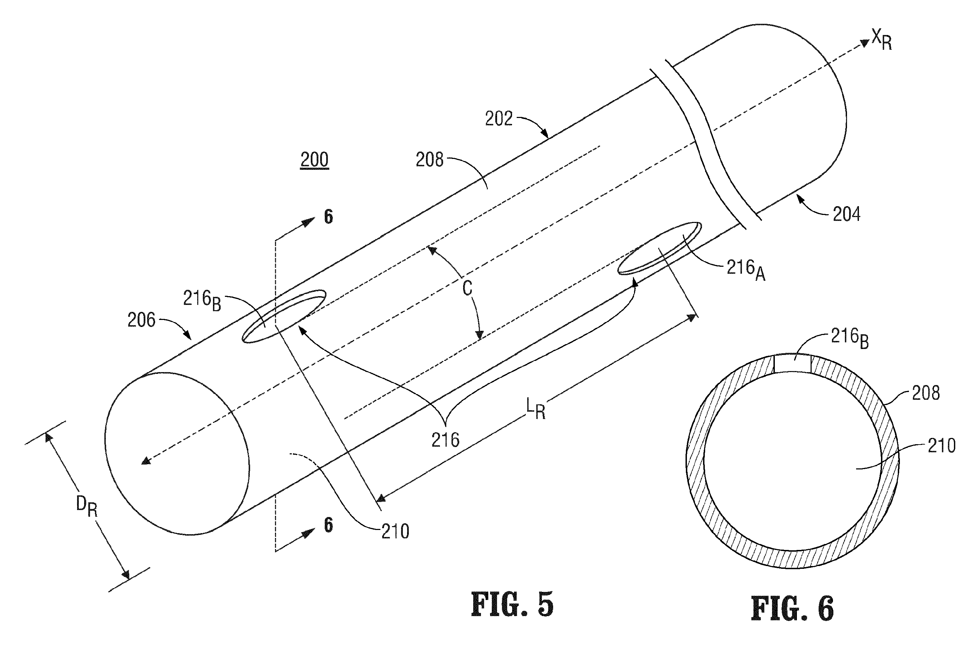

With reference now to FIGS. 1 and 6, the regulator 200 is dimensioned for insertion into the balloon lumen 110 that extends through the catheter body 102. The regulator 200 may be formed, such as by extrusion, from one or more materials having sufficient rigidity to facilitate manipulation of the regulator 200 with respect to the catheter 100.

The regulator 200 includes a regulator body 202 having a proximal end portion 204 and a distal end portion 206, and defining a longitudinal axis X_{R} therebetween. The proximal end portion 204 of the regulator 200 is connectable into fluid communication with the fluid source 300. The proximal end portion 204 of the regulator 200 extends proximally beyond the proximal end portion 104 of the catheter body 102 when the regulator 200 is positioned in the balloon lumen 110 of the catheter body 102 such that one or more openings 216 defined by the regulator 200 are aligned with one or more openings 116 defined by the catheter 100. As described in further detail below, the user can rotate the regulator 200 within the balloon lumen 110 and/or longitudinally moving the regulator 200 within the balloon lumen 110 with respect to the lumen axis X_{B} to arrange the regulator at a defined orientation with respect to the catheter 100.

The distal end portion 206 of the regulator body 202 is closed such that fluid communicated into the regulator 200 is inhibited from moving beyond the distal end portion 206 and fluid pressure builds in the regulator body 202 as fluid is introduced from the fluid source 300. The distal end portion 206 may include a monolithic component of the regulator body 202. Additionally or alternatively, the distal end portion 206 may include a cap (not shown) secured to the regulator body 202, e.g., through welding or the use of an adhesive.

The regulator body 202 includes wall 208 defining passage 210 extending from the proximal end portion 204 to the distal end portion 206. The passage 210 receives fluid communicated from the fluid source 300. The wall 208 of the regulator body 202 has an outer cross-sectional dimension D_{R} transverse to the longitudinal axis X_{R} of the regulator 200. The outer cross-sectional dimension D_{R} is approximately equal to the inner transverse cross-sectional dimension D_{L2} (FIG. 2) defined by the balloon lumen 110 such that the outer wall 208 of the regulator body 202 forms a substantially fluid tight seal with an inner surface 118 of the catheter 100 defining the balloon lumen 110.

The outer cross-sectional dimension D_{R} of the wall 208 of the regulator body 202 is shown as being uniform from the proximal end portion 204 to the distal end portion 206 of the regulator body 202.

The wall 208 of the regulator body 202 is generally smooth to facilitate manipulation of the regulator 200 within the balloon lumen 110 (FIG. 2) of the catheter 100, and defines one or more openings 216. The outer surface may include a lubricious coating such as silicone. The opening(s) 216 correspond in number to the orifice(s) 116 defined by the catheter body 102 and are in fluid communication with the passage 210. Although the regulator 200 is shown as including a pair of openings 216_{A} and 216_{B} in FIG. 5 such that each opening 216_{A}, 216_{B} corresponds to each of the orifices 116_{A} and 116_{B} defined by the catheter body 102, it should be appreciated that the number of openings 216 defined by the regulator body 202 may vary dependent upon the number of orifice 116 and balloon members 112 associated with the catheter 100, and/or with the particular requirements of the procedure in which the catheter 100 and the regulator 200 are used.

The openings 216_{A} and 216_{B} are spaced apart from one another along the longitudinal axis X_{R} of the regulator 200 by an axial distance L_{R} approximately equal to the axial distance Lc (FIG. 2) separating the orifices 116_{A} and 116_{B}. The openings 216_{A} and 216_{B} are alignable with the orifices 116_{A} and 116_{B}, respectively, via axial and/or rotational manipulation of the regulator 200 within the balloon lumen 110.

During use of the regulator 200 (FIG. 5) in conjunction with the catheter 100 (FIG. 2), e.g., depending upon the orientation of the regulator 200 relative to the catheter 100, fluid communicated into the passage 210 of the regulator 200 will exit the passage 210 through one of the openings 216_{A}, 216_{B}, and enters a respective balloon members 112_{A}, 112_{B}.

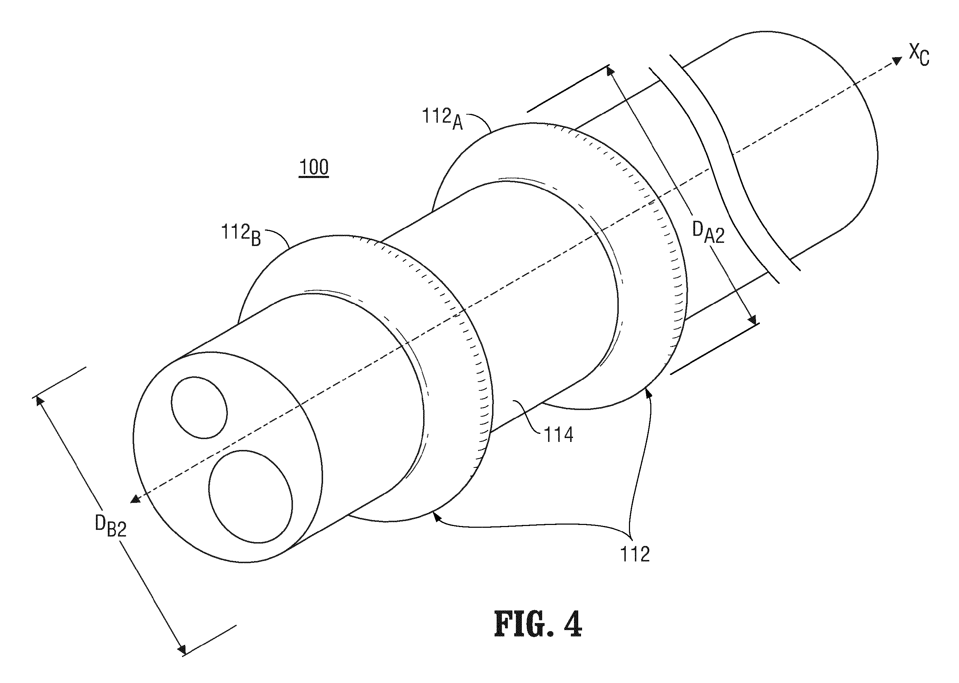

Referring now to FIGS. 5-7D, prior to axial and circumferential alignment of the orifices 116_{A}, 116_{B} of the catheter body 102 and the openings 216_{A}, 216_{B} of the regulator body 202, respectively, fluid communicated into the passage 210 of the regulator 200 is maintained within the passage 210 by the seal formed between the wall 208 of the regulator body 202 and the inner surface 118 (FIG. 2) of the balloon lumen 110 of the catheter 100 and is, thus, prevented from escaping through the orifices 116_{A}, 116_{B}. Upon axial and circumferential alignment of the orifice 116_{A} with the opening 216_{A} via rotation and/or axial positioning of the regulator 200 in relation to the balloon lumen 110 of the catheter 100, fluid communicated into the passage 210 of the regulator 200 exits through the opening 216_{A} and enters the interior volume of the balloon 112_{A} through the orifice 116_{A}. Such introduction of fluid into the interior of the balloon member 112_{A} transitions the balloon 112_{A} from the uninflated condition (FIG. 2) to the at least partially inflated condition (FIG. 4). Likewise, upon axial and circumferential alignment of the orifice 116_{B} with the opening 216_{B} fluid exits the passage 210 of the regulator 200 through the opening 216_{B} and enters the interior volume of the balloon 112_{B} through the orifice 116_{B}. Such introduction of fluid into the interior of the balloon member 112_{B} transitions the balloon 112_{B} from the uninflated condition (FIG. 2) into the at least partially inflated condition (FIG. 4).

The openings 216_{A}, 216_{B} in the wall 208 of the regulator body 202 are offset from each other by a circumferential distance C. It should be appreciated that the dimensional relationship between the distance C and the cross-sectional dimension Do (FIG. 3) defined by the orifices 116_{A}, 116_{B} of the catheter body 102 determines whether the balloons 112_{A}, 112_{B} are independently or simultaneously expandable. For example, if the distance C (FIG. 5) is greater than the cross-sectional dimension Do (FIG. 3) defined by the orifices orifice 116_{A}, 116_{B}, the regulator 200 may be rotated within the balloon lumen 110 such that the opening 216_{A} and the orifice 116_{A} are aligned while the opening 216_{B} and the orifice 116_{B} are out of alignment. With the opening 216_{A} and the orifice 116_{A} aligned while the opening 216_{B} is out of alignment with the orifice 116_{B}, fluid communicated into the passage 210 of the regulator 200 exits the passage 210 through the opening 216_{A} and moves through the orifice 116_{A} into the interior volume of the balloon 112_{A} to inflate the balloon member 112_{A} while the balloon 112 remains uninflated. At a separate point in time, such as after the balloon 112_{A} has been inflated, the regulator 200 may be rotated within the balloon lumen 110 such that the opening 216_{B} and the orifice 116_{B} are aligned while the opening 216_{A} and the orifice 116_{A} are out of alignment. With the opening 216_{B} and the orifice 116_{B} aligned while the opening 216_{A} and the orifice 116_{A} are unaligned, fluid communicated into the passage 210 of the regulator 200 exits the passage 210 through the opening 216_{B} and moves through the orifice 116_{B} into the interior volume of the balloon 112_{B} to inflate the balloon 112_{A}. As another example, if the distance C is less than the cross-sectional dimension Do defined by the orifices orifice 116_{A} and 116_{B}, the regulator 200 may be rotated within the balloon lumen 110 such that portions of the openings 216_{A}, 216_{B} are brought simultaneously into alignment with portions of the orifices 116_{A}, 116_{B}, respectively, as seen in FIG. 5B. The simultaneous alignment of the openings 216_{A}, 216_{B} with the respective orifices 116_{A}, 116_{B} permits fluid communicated into the passage 210 of the regulator 200 to exit the passage 210 through the openings 216_{A}, 216_{B} and move through the orifices 116_{A}, 116_{B} into the respective balloons 112_{A}, 112_{B} to inflate the balloons 112_{A}, 112_{B}.

With reference now to FIGS. 1-7D, an exemplary method of using the system 1000 during a medical procedure includes inserting the catheter 100 into a patient's vasculature, for example, the blood vessel V, while the balloons 112_{A}, 112_{B} are in an inflated condition, and advancing the catheter 100 until the catheter 100 is positioned in a location suitable for performance of the medical procedure.

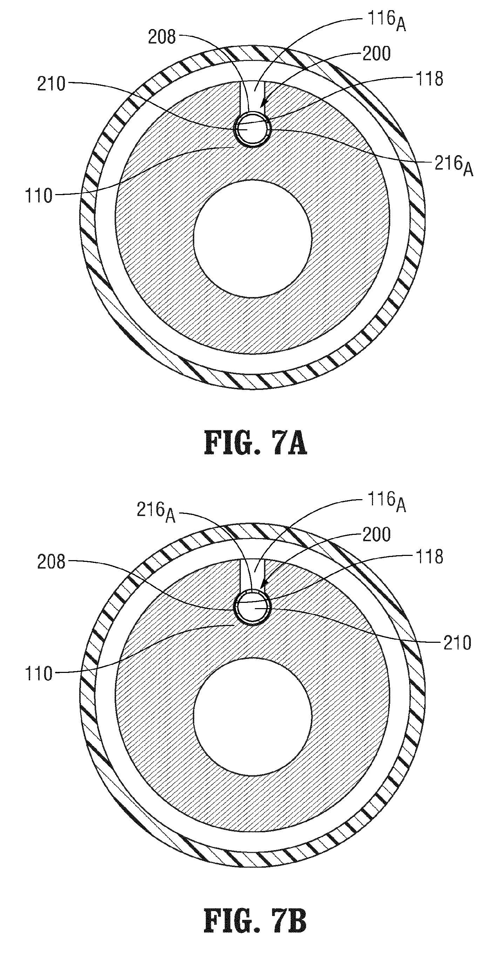

The regulator 200 is inserted into the balloon lumen 110 (FIG. 2) and oriented such that the openings 216_{A}, 216_{B} formed in the outer wall 208 of the regulator body 202 and the orifices 116_{A}, 116_{B} formed in the outer surface 114 of the catheter 100 are axially and/or circumferentially misaligned as shown, for example, in FIG. 7A. Fluid may be communicated into the passage 210 extending through the regulator 200 from the fluid source 300, via the proximal end portion 204, and the misalignment of the openings 216_{A}, 216_{B} and the respective openings 116_{A}, 116_{B} can prevent the fluid from being communicated from the passage 210 of the regulator 200 to the internal volume of the balloon members 112_{A}, 112_{B}.

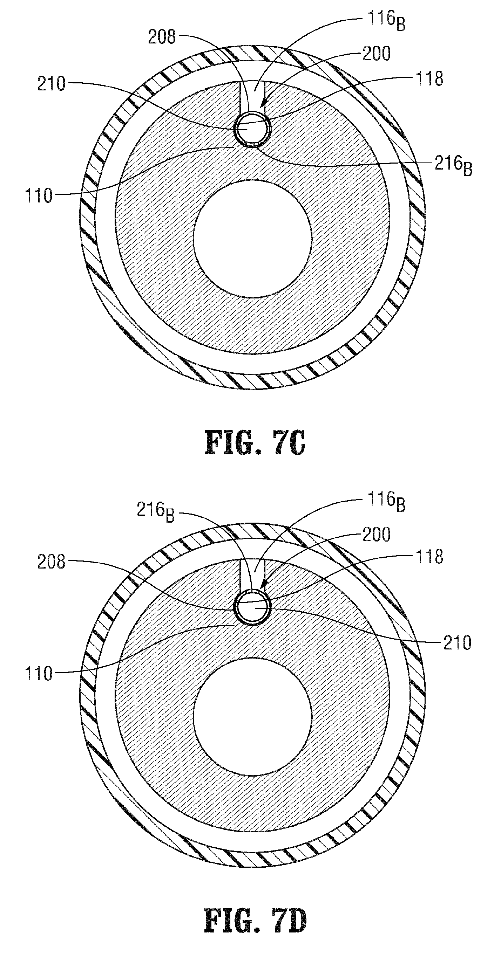

The regulator 200 can be manipulated within the balloon lumen 110 to align the opening 216_{A} with the orifice 116_{A} (see, e.g., FIG. 7B), while the opening 216_{B} and the orifice 116_{B} to remain out of alignment (see, e.g., FIG. 7C) such that the orifice 116_{B} is sealed by the regulator body 202. With the opening 216_{A} aligned with the orifice 116_{A}, fluid can exit the passage 210 and enter the balloon 112_{A} to move the balloon member 112_{A} from the uninflated condition (FIG. 2) and into the inflated condition (FIG. 4), while the balloon 112_{B} remains in the uninflated condition. Additionally or alternatively, the regulator 200 can be manipulated within the balloon lumen 110 to align the opening 216_{B} and the orifice 116_{B} (see, e.g., FIG. 7D), and cause misalignment between the opening 216_{A} and the orifice 116_{A} (see, e.g., FIG. 7A) such that the orifice 116_{A} is sealed by the regulator body 202, causing any fluid in the balloon member 112_{A} to remain within the balloon member 112_{A}. With the opening 216_{B} and the orifice 116_{B} aligned, fluid can exit the passage 210 and enter the balloon member 112_{B} to move the balloon member 112_{B} from the uninflated condition (FIG. 2) into the second expanded condition (FIG. 4). During the procedure, the regulator 200 may be rotated and/or moved axially within the balloon lumen 110 to misalign both openings 216_{A}, 216_{B} relative to respective orifices 116_{A}, 116_{B} to prevent further introduction of fluids within the balloons 112_{A}, 112_{B}. For example, the regulator 200 may be rotated and/or moved axially within the balloon lumen 110 when one or both of the balloons 112_{A}, 112_{B} reaches a target inflation pressure. In this misaligned orientation, a seal between the outer surface of the regulator body 202 and the inner surface 118 of the balloon lumen 110 may be prevent fluid from escaping the balloons 112_{A}, 112_{B} through the openings 216_{A}, 216_{B}. Thus, the balloons 112_{A}, 112_{B} may remain in the respective inflated conditions.

The balloons 112_{A}, 112_{B} may, for example, be expanded to center the catheter 100 within the blood vessel V (FIG. 1). Centering the catheter 100 within the blood vessel V can facilitate symmetrical spacing of the catheter 100 from the internal wall W of the blood vessel V. Such symmetrical spacing of the catheter 100 from the internal wall W of the blood vessel V may facilitate, for example, increased efficacy in the treatment of an occlusion (not shown) present within the blood vessel V.

With the balloon 112_{A} and/or the balloon 112_{B} in the inflated condition shown in FIG. 4, a surgical instrument, such as a thrombectomy catheter (not shown), may be inserted into, and advanced through, the main lumen 108 extending through the catheter 100 to perform a thrombectomy procedure.

While certain embodiments have been described, other embodiments are possible.

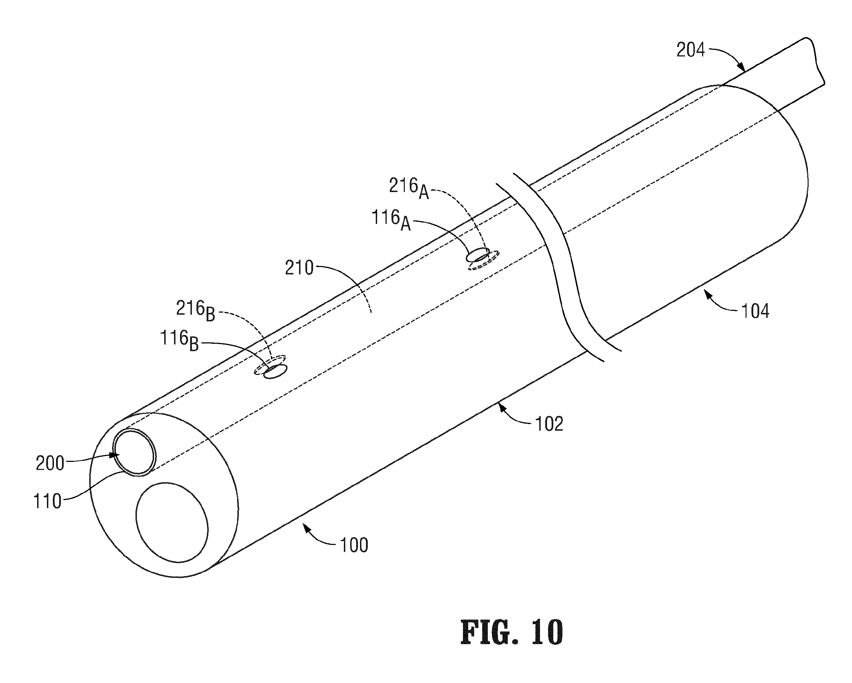

For example, while the fluids have been described as passing through the passage 210 to inflate balloons 112_{A}, 112_{B} separately, other configurations are additionally or alternatively possible. For example, with reference to FIG. 10, fluids may enter the passage 210 and communicate with the internal volumes of the balloon members 112_{A}, 112_{B} substantially simultaneously. For example, the regulator 200 of FIG. 10 can be manipulated within the balloon lumen 110 to cause simultaneous alignment between the openings 216_{A}, 216_{B} and the orifices 116_{A}, 116_{B}, permitting fluid to exit the passage 210, and simultaneously enter the balloon members 112_{A}, 112_{B}.

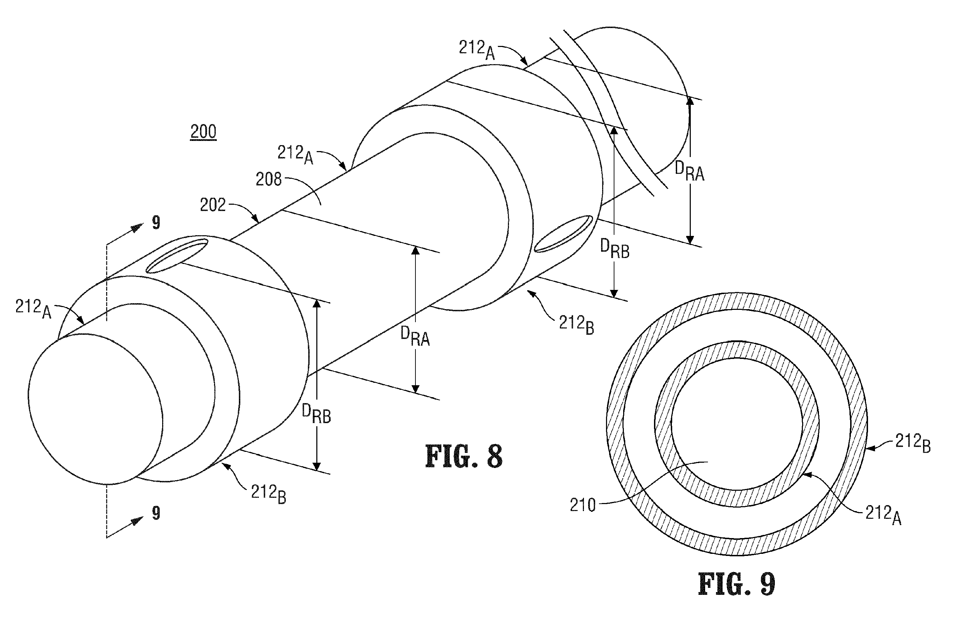

As yet another example, while the wall 208 of the regulator body 202 has been shown as having a uniform outer cross-sectional dimension, other configurations are additionally or alternatively possible. For example, as shown in FIGS. 8 and 9, the outer wall 208 of the regulator body 202 may include first sections 212_{A} defining outer cross-sectional dimensions D_{RA}, and second sections 212_{B} defining larger outer cross-sectional dimensions D_{RB}. The outer cross-sectional dimension D_{RB} may be approximately equal to the inner cross-sectional dimension D_{L2} (FIG. 2) defined by the balloon lumen 110 of the catheter 100 such that each of the second sections 212_{B} forms a substantially fluid tight seal with the inner wall 118 defining the balloon lumen 110. This arrangement may, for example, reduce the surface area of the regulator 200 contacting the inner wall of the balloon lumen 110, reducing friction and facilitating manipulation (e.g., rotation and/or axial movement) of the regulator 200 within the balloon lumen 110.

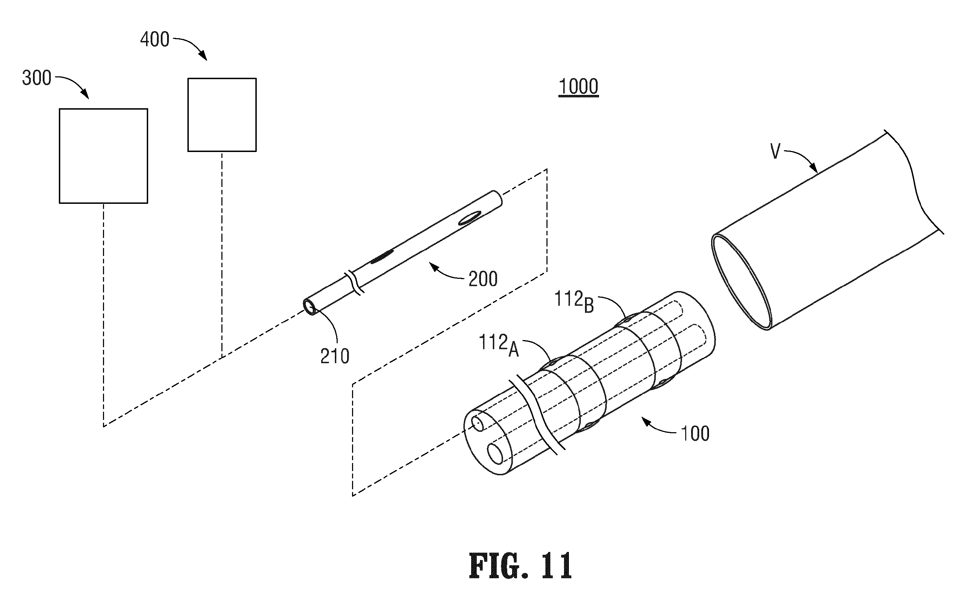

The balloon catheter system 1000 may also include a sensor 400 to measure the pressure of the fluid introduced into the regulator 200 from the source of fluid 300. As seen in FIG. 11, the sensor 400 is fluid communication with the passage 210 extending through the regulator 200.

During use, manipulation of the regulator 200 within the catheter 100 may be based, at least in part, upon the measured pressure of the fluid communicated into the regulator 200 from the fluid source 300. For example, the regulator 200 may be oriented within the catheter 100 to permit fluid flow into the balloon 112_{A} and/or the balloon 112_{B} in the manner discussed above until a predetermined pressure is measured by the sensor 400. Thereafter, the regulator 200 may be re-oriented within the catheter 100 to interrupt fluid flow into the balloon 112_{A} and/or the balloon 112_{B}. Additionally, the sensor 400 may generate an audible and/or visual signal to communicate to the user that the predetermined pressure has been measured.

Persons skilled in the art will understand that the devices and methods specifically described herein, and illustrated in the accompanying drawings, are non-limiting, exemplary embodiments of the present disclosure, and that the elements and features illustrated or described in connection with one exemplary embodiment may be combined with those of another embodiment without departing from the scope of the present disclosure.

As well, one skilled in the art will appreciate further features and advantages of the devices and methods described herein based on the above-described embodiments and the claims. Accordingly, the present disclosure is not limited by what has been particularly shown and described.

A method of controlling inflation of a balloon catheter, the method comprising:
positioning at least a distal end portion of a regulator within a balloon lumen defined by a catheter;
introducing fluid into a proximal end portion of a passage defined by the regulator, the passage extending from the proximal end portion to the distal end portion of the regulator, the distal end portion of the regulator having an outer surface defining at least one opening in fluid communication with the passage; and
moving the at least one opening of the regulator within the balloon lumen to control fluid communication between the passage and the at least one balloon.

The method described above, wherein moving the at least one opening of the regulator comprises aligning the at least one opening with at least one orifice defined by an outer surface of the catheter, the at least one orifice in fluid communication with the balloon such that fluid introduced into the passage of the regulator flows into a volume defined by the balloon.

The method described above, wherein aligning the at least one opening with the at least one orifice defined by the outer surface of the catheter comprises rotating the regulator about a longitudinal axis of the balloon lumen.

The method described above, wherein moving the at least one opening of the regulator comprises misaligning the at least one opening and the at least one orifice to inhibit the flow of fluid from the passage of the regulator into the volume defined by the balloon.

The method described above, further comprising measuring pressure of the fluid introduced into the proximal end portion of the passage of the regulator, wherein moving the at least one opening of the regulator is based at least in part on the measured pressure of the fluid.

## Claims

1. A system for use in a medical procedure, the system comprising:
a catheter defining a balloon lumen;
at least one balloon secured to an outer surface of the catheter; and
a regulator at least partially disposed within the balloon lumen, the regulator including a proximal end portion and a distal end portion, the regulator defining a passage extending from the proximal end portion to the distal end portion, the distal end portion of the regulator having an outer surface defining at least one opening in fluid communication with the passage,
wherein the at least one opening of the regulator is movable within the balloon lumen to control fluid communication between the passage and the at least one balloon.

2. The system of claim 1, wherein a portion of the catheter defining the balloon lumen forms a substantially fluid-tight seal with a portion of the regulator adjacent the at least one opening.

3. The system of claim 1, wherein the at least one opening is movable along a longitudinal axis of the balloon lumen to control fluid communication between the passage and the at least one balloon.

4. The system of claim 1, wherein the at least one opening is a plurality of openings axially spaced from one another along a longitudinal axis of the balloon lumen of the catheter.

5. The system of claim 1, wherein the at least one opening is rotatable about a longitudinal axis of the balloon lumen to control fluid communication between the passage and the at least one balloon.

6. The system of claim 1, wherein the at least one opening is a plurality of openings circumferentially spaced from one another along the outer surface of the regulator.

7. The system of claim 1, wherein the at least one balloon spans a circumference of the outer surface of the catheter body.

8. The system of claim 1, wherein the at least one balloon comprises a proximal balloon and a distal balloon, the proximal and distal balloons axially spaced from one another along a longitudinal axis of the balloon lumen.

9. The system of claim 1, wherein the at least one balloon comprises a first balloon and a second balloon, and the at least one opening is movable within the balloon lumen to establish fluid communication between the passage of the regulator and one of the first and second balloons while fluidly isolating the passage of the regulator from the other one of the at first and second balloons.

10. The system of claim 1, wherein the distal end portion of the regulator includes a closed end distal to the at least one opening.

11. The system of claim 1, wherein an outer, transverse cross-section of the regulator is uniform from the proximal end portion to the distal end portion.

12. The system of claim 1, wherein an outer, transverse cross-section of the regulator is largest adjacent the at least one opening.

13. The system of claim 1, wherein the catheter further defines a main lumen substantially parallel to the balloon lumen.

14. The system of claim 13, wherein a transverse cross-sectional area of the main lumen is larger than a transverse cross-sectional area of the balloon lumen.

15. The system of claim 1, wherein the outer surface of the catheter defines at least one orifice in fluid communication with the at least one balloon, and the at least one opening of the regulator is movable within the balloon lumen to control fluid communication between the passage and the at least one orifice defined by the outer surface of the catheter.
